# EUROPEAN PATENT APPLICATION

(11) **EP 3 843 102 A1**
(43) Date of publication of application: **30.06.2021**
(21) Application number: 19852969.5
(22) Date of filing: 22.07.2019
(51) Int. Cl.: G16H 20/00

(54) **HEALTH MANAGEMENT ASSISTANCE DEVICE, HEALTH MANAGEMENT ASSISTANCE METHOD, HEALTH MANAGEMENT ASSISTANCE TERMINAL, AND PROGRAM**

(30) Priority: 23.08.2018 JP 2018156033
(71) Applicant: NEC Solution Innovators, Ltd., Koto-ku, Tokyo 136-8627 (JP)
(72) Inventor: YOSHIDA Yoshihito, Tokyo 136-8627 (JP); CHIBA Takamasa, Fuchu-shi, Tokyo 183-8502 (JP); OCHIAI Toshifumi, Kawasaki-shi, Kanagawa 211-8601 (JP); KOBAYASHI Hirokazu, Tokyo 108-8001 (JP); YASUMOTO Saki, Tokyo 108-8001 (JP)
(74) Representative: Betten & Resch
(86) International application number: PCT/JP2019/028609
(87) International publication number: WO 2020/039813

(57) **Abstract**

The present invention provides a health management assistance apparatus, a health management assistance method, and a health management assistance terminal that allow a user to easily realize health management. The health management assistance apparatus of the present invention includes: an acquisition unit that acquires purchased food information of a user, an extraction unit that extracts nutritional composition information of a purchased food from the purchased food information; an estimation unit that estimates a future health status of the user from the nutritional composition information; and an output unit that outputs the future health status of the user.

## Description

### TECHNICAL FIELD

The present invention relates to a health management assistance apparatus, a health management assistance method, a health management assistance terminal, and a program.

### BACKGROUND ART

Medical expenditures account for 41% of the national budget and are expected to exceed 50% by 2030. Against this backdrop, efforts to extend healthy life expectancy and healthcare products and services to prevent illness have been offered. In addition, with the development of ICT technology, health management applications and systems using ICT technology have been utilized.

As a healthcare service utilizing the ICT technology, Patent Literature 1 discloses a diet management apparatus that determines a diet tendency of a user estimated from past contents of the meal of the user, and can check the ingredients according to the diet tendency of the user even when the user forgets to input contents of the meal. Patent Literature 2 discloses a diet management assistance system that analyzes a nutritional composition from an image of a meal taken in by a person requiring diet management and accumulates the obtained nutritional composition, and further transmits an analysis result and an advice to the person requiring diet management. Patent Literature 3 discloses a method of recording nutritional composition information of a food taken in by a meal, analyzing the nutritional composition in the recorded information of the meal, and presenting an excess or an insufficiency of a necessary nutritional composition.

### Citation List

### Patent Literature

Patent Literature 1: Japanese Patent No. 5843382
Patent Literature 2: Japanese Patent No. 4989240
Patent Literature 3: Japanese Patent No. 4987042

### SUMMARY OF INVENTION

### Technical Problem

When performing health management, the user inputs purchased food information and the like. Then, the health management assistance apparatus analyzes the purchased food information and outputs the daily nutritional status of the user. However, daily nutritional status does not change significantly. Therefore, there is a problem that, even if the user acquires the daily nutritional status, it is difficult to grasp how the health status is changing, that is, whether health management is well performed, and it is difficult to realize the health management.

With the foregoing in mind, it is an object of the present invention to provide a health management assistance apparatus, a health management assistance method, and a health management assistance terminal that allow a user to easily realize health management.

### Solution to Problem

In order to achieve the aforementioned object, the present invention provides a health management assistance apparatus (also referred to as an "assistance apparatus" hereinafter), including: an acquisition unit that acquires purchased food information of a user, an extraction unit that extracts nutritional composition information of a purchased food from the purchased food information; an estimation unit that estimates a future health status of the user from the nutritional composition information; and an output unit that outputs the future health status of the user.

The present invention also provides a health management assistance method (also referred to as an "assistance method" hereinafter), including: an acquisition step of acquiring purchased food information of a user, an extraction step of extracting nutritional composition information of a purchased food from the purchased food information; an estimation step of estimating a future health status of the user from the nutritional composition information; and an output step of outputting the future health status of the user.

The present invention also provides a health management assistance terminal (also referred to as an "assistance terminal" hereinafter), including: an input unit that inputs purchased food information of a user; a communication unit configured to communicate with the health management assistance apparatus according to the present invention that estimates a future health status of the user from the purchased food information of the user; and a display unit that displays the future health status of the user.

### Advantageous Effects of Invention

The present invention allows a user to easily realize health management.

### BRIEF DESCRIPTION OF DRAWINGS

[FIG. 1] FIG. 1 is a block diagram showing the configuration of an example of an assistance apparatus, an assistance terminal, and an assistance system that includes the assistance apparatus and the assistance terminal according to the first example embodiment.
[FIG. 2] FIG. 2 is a block diagram showing an example of the hardware configuration of the assistance apparatus according to the first example embodiment.
[FIG. 3] FIG. 3 is a block diagram showing an example of the hardware configuration of the assistance terminal according to the first example embodiment.
[FIG. 4] FIG. 4 is a flowchart showing the configuration of an example of an assistance method and a program according to the first example embodiment.
[FIG. 5] FIG. 5 is a block diagram showing the configuration of an example of an assistance apparatus according to the second example embodiment.
[FIG. 6] FIG. 6 is a flowchart showing an assistance method and a program according to the second example embodiment.
[FIGs. 7A to 7C] FIGs. 7A to 7C are diagrams showing an example of an image displayed on the display unit of the assistance terminal according to the second example embodiment.
[FIG. 8] FIG. 8 is a block diagram showing an example of the configuration of an assistance apparatus according to the third example embodiment.
[FIG. 9] FIG. 9 is a flowchart showing an assistance method and a program according to the third example embodiment.

### DESCRIPTION OF EXAMPLE EMBODIMENTS

The assistance apparatus of the present invention may include: an image creation unit that creates a character image representing an individual of the user from the future health status of the user, wherein the output unit may output the character image as the future health status of the user. According to this aspect, by outputting a health status, which is difficult to disclose to someone other than the user such as a family member or the like, using a character image, it becomes easy to disclose the health status to a third party such as the family member or the like. Therefore, according to this aspect, a third party can intervene in the health management of the user, which promotes the health management of the user.

The assistance apparatus of the present invention may include: a control information acquisition unit that acquires control information for controlling at least one selected from the group consisting of an action of a character, a facial expression of the character, a background image of the character image, and a display position of the character image associated with the health status based on the future health status of the user, wherein the output unit may output the control information. According to this aspect, by changing the action of the character, the facial expression of the character image, and the background image of the character image, the future health status of the user can be displayed more effectively. Therefore, according to this aspect, it is possible to make it easier for the user to realize health management.

In the assistance apparatus of the present invention, the estimation unit may extract an excessive or insufficient nutritional composition from the nutritional composition information and a nutritional composition reference value of the user, and may estimate the future health status of the user from the excessive or insufficient nutritional composition in the nutritional composition information and a reference composition of the health status. According to this aspect, since the future health status of the user is estimated with reference to the excessive or insufficient nutritional composition taken in by the user, it is possible to estimate the future health status of the user with better accuracy. Therefore, according to this aspect, it is possible to give the user a sense of trust in the information of the health management, which promotes the health management of the user.

In the assistance apparatus of the present invention, the acquisition unit may acquire attribute information of the user, and the estimation unit may calculate the nutritional composition reference value of the user from the attribute information of the user and a nutritional composition intake reference value for each attribute. According to this aspect, since the nutritional composition reference value suitable for the user can be set, it is possible to estimate the future health status of the user with better accuracy. Therefore, according to this aspect, it is possible to give the user a sense of trust in the information of the health management, which promotes the health management of the user.

In the assistance apparatus of the present invention, the acquisition unit may acquire the attribute information of the user. In this case, in the assistance apparatus of the present invention, preferably, the attribute information of the user includes an age of the user, the apparatus includes an age determination unit that determines whether the age of the user satisfies an age condition, when the age determination unit determines that the age of the user satisfies the age condition, the estimation unit extracts an excessive nutritional composition by comparing the nutritional composition information with the nutritional composition reference value of the user, and estimates the future health status of the user from the excessive nutritional composition in the nutritional composition information and a reference composition of a health status. Further, in the assistance apparatus of the present invention, preferably, the attribute information of the user includes an age of the user, the apparatus includes an age determination unit that determines whether the age of the user satisfies an age condition, when the age determination unit determines that the age of the user does not satisfy the age condition, the estimation unit extracts an insufficient nutritional composition by comparing the nutritional composition information with the nutritional composition reference value of the user, and estimates the future health status of the user from the insufficient nutritional composition in the nutritional composition information and a reference composition of a health status. According to this aspect, since information on more important nutritional composition is extracted depending on the age of the user and the future health status of the user is estimated based on the extracted nutritional composition, it is possible to estimate the future health status of the user with better accuracy. Therefore, according to this aspect, it is possible to give the user a sense of trust in the information of the health management, which promotes the health management of the user.

In the assistance apparatus of the present invention, the estimation unit may estimate a current health status of the user from the nutritional composition information. In this case, in the assistance apparatus of the present invention, the output unit preferably outputs the current health status of the user. According to this aspect, the user can check changes in the health status by comparing the current health status of the user with the future health status of the user. Therefore, according to this aspect, it is possible to make it easier for the user to realize health management.

In the assistance apparatus of the present invention, the output unit may output the future health status of the user to a terminal of the user or to a terminal different from the terminal of the user.

In the assistance apparatus of the present invention, the extraction unit may acquire the nutritional composition information of the purchased food from outside the apparatus.

The assistance apparatus of the present invention may be a server.

The assistance method of the present invention may include an image creation step of creating a character image representing an individual of the user from the future health status of the user, wherein in the output step, the character image may be output as the future health status of the user. According to this aspect, by outputting a health status, which is difficult to disclose to someone other than the user such as a family member or the like, using a character image, it becomes easy to disclose the health status to a third party such as the family member or the like. Therefore, according to this aspect, a third party can intervene in the health management of the user, which promotes the health management of the user.

The assistance method of the present invention may include a control information acquisition step of acquiring control information for controlling at least one selected from the group consisting of an action of the character, a facial expression of the character, a background image of the character image, and a display position of the character image associated with the health status based on the future health status of the user, wherein in the output step, the control information may be output. According to this aspect, by changing the action of the character, the facial expression of the character image, and the background image of the character image, the future health status of the user can be displayed more effectively. Therefore, according to this aspect, it is possible to make it easier for the user to realize health management.

In the assistance method of the present invention, in the estimation step, an excessive or insufficient nutritional composition may be extracted from the nutritional composition information and a nutritional composition reference value of the user, and the future health status of the user may be estimated from the excessive or insufficient nutritional composition in the nutritional composition information and a reference composition of a health status. According to this aspect, since the future health status of the user is estimated with reference to the excessive or insufficient nutritional composition taken in by the user, it is possible to estimate the future health status of the user with better accuracy. Therefore, according to this aspect, it is possible to give the user a sense of trust in the information of the health management, which promotes the health management of the user.

In the assistance method of the present invention, in the acquisition step, attribute information of the user may be acquired, and, in the estimation step, the nutritional composition reference value of the user may be calculated from the attribute information of the user and a nutritional composition intake reference value for each attribute. According to this aspect, since the nutritional composition reference value suitable for the user can be set, it is possible to estimate the future health status of the user with better accuracy. Therefore, according to this aspect, it is possible to give the user a sense of trust in the information of the health management, which promotes the health management of the user.

In the assistance method of the present invention, in the acquisition step, the attribute information of the user may be acquired. In this case, in the assistance method of the present invention, preferably, the attribute information of the user includes an age of the user, the method includes an age determination step of determining whether the age of the user satisfies an age condition, when the age of the user is determined to satisfy the age condition in the age determination step, in the estimation step, an excessive nutritional composition is extracted from the nutritional composition information and the nutritional composition reference value of the user, and the future health status of the user is estimated from the excessive nutritional composition in the nutritional composition information and a reference composition of a health status. Further, in the assistance method of the present invention, preferably, the attribute information of the user includes an age of the user, the method includes an age determination step of determining whether the age of the user satisfies an age condition, when the age of the user is determined not to satisfy the age condition in the age determination step, in the estimation step, an insufficient nutritional composition is extracted from the nutritional composition information and the nutritional composition reference value of the user, and the future health status of the user is estimated from the insufficient nutritional composition in the nutritional composition information and a reference composition of a health status. According to this aspect, since information on more important nutritional composition is extracted depending on the age of the user and the future health status of the user is estimated based on the extracted nutritional composition, it is possible to estimate the future health status of the user with better accuracy. Therefore, according to this aspect, it is possible to give the user a sense of trust in the information of the health management, which promotes the health management of the user.

In the assistance method of the present invention, a current health status of the user may be estimated from the nutritional composition information in the estimation step. In the assistance method of the present invention, preferably, the current health status of the user is output in the output step. According to this aspect, the user can check changes in the health status by comparing the current health status of the user with the future health status of the user. Therefore, according to this aspect, it is possible to make it easier for the user to realize health management.

In the assistance method of the present invention, in the output step, the future health status of the user may be output to a terminal of the user or to a terminal different from the terminal of the user.

In the assistance method of the present invention, in the extraction step, the nutritional composition information of the purchased food may be acquired from outside the apparatus.

The program of the present invention can execute the following processing on a computer: acquisition processing of acquiring purchased food information of a user; extraction processing of extracting nutritional composition information of the purchased food from the purchased food information; estimation processing of estimating a future health status of the user from the nutritional composition information; and output processing of outputting the future health status of the user.

The computer readable recording medium of the present invention records the program of the present invention.

The health management assistance system (also referred to as an "assistance system" hereinafter) of the present invention includes: a terminal; and a server, wherein the terminal and the server are connectable via a communication line network outside the system, the terminal includes: an input unit that inputs purchased food information of a user; a communication unit configured to communicate with the server; and a display unit that displays a future health status of the user, and the server includes: an acquisition unit that acquires the purchased food information of the user; an extraction unit that extracts nutritional composition information of the purchased food from the purchased food information; an estimation unit that estimates the future health status of the user from the nutritional composition information; and an output unit that outputs the future health status of the user.

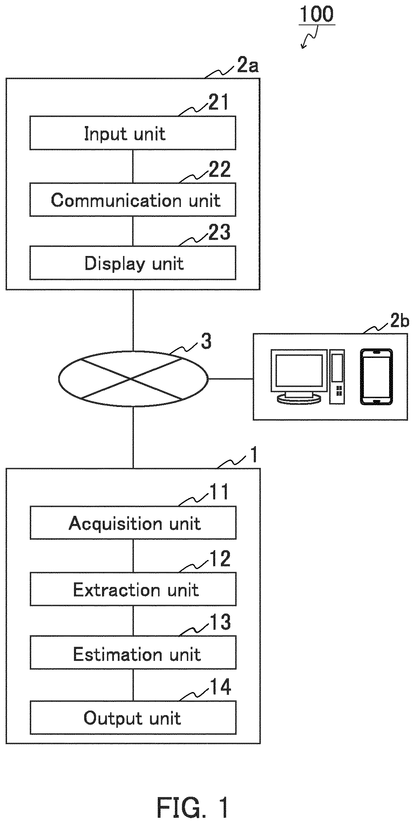

Example embodiments of the present invention will be described with reference to FIGs. 1 to 9. It is to be noted, however, that the present invention is by no means limited or restricted by the following example embodiments. Hereinafter, in FIGs. 1 to 9, identical parts are indicated with identical reference signs. Regarding the descriptions of the example embodiments, reference can be made to one another unless otherwise stated. Furthermore, the configurations of the example embodiments can be combined unless otherwise stated.

### First example embodiment

The present example embodiment is an example of an assistance system including an example of the assistance apparatus and the assistance terminal of the present invention. FIG. 1 is a block diagram showing the configuration of an example of an assistance system 100 including an example of an assistance apparatus 1 and assistance terminal 2a, 2b of the present example embodiment. As shown in FIG. 1, the assistance system 100 includes the assistance apparatus 1, the assistance terminal 2a of the user, and the assistance terminal 2b different from the terminal of the user (the assistance terminal of someone other than the user). Further, as shown in FIG. 1, the assistance apparatus 1 includes an acquisition unit 11, an extraction unit 12, an estimation unit 13, and an output unit 14. The assistance terminal 2a includes an input unit 21, a communication unit 22, and a display unit 23. Since the assistance terminal 2b has the same configuration as the assistance terminal 2a, the description of each unit is omitted. As shown in FIG. 1, the assistance apparatus 1 is connectable to the assistance terminal 2a, 2b via a communication line network 3. The assistance apparatus 1 of the present example embodiment may be incorporated in a server as a system. The assistance apparatus 1 of the present example embodiment may be a personal computer (PC) in which the program of the present invention is installed. Although it is not shown, the assistance apparatus 1 is connectable to the external terminal of the system administrator via the communication line network 3, and the system administrator may manage the assistance apparatus 1 from the external terminal.

The communication line network 3 is not particularly limited, and a known network can be used, and may be, for example, a wired network or a wireless network. Examples of the communication line network 3 include an Internet line, a WWW (World Wide Web), a telephone line, a LAN (Local Area Network), and a WiFi (Wireless Fidelity).

Examples of the assistance terminal 2a, 2b include PCs; mobile terminals such as a mobile phone, a smart phone, a tablet, and the like; and wearable terminals such as a smart watch, a smart glass, and the like. The assistance terminal 2a, 2b may be provided with, for example, imaging units such as a camera, a scanner, and the like; IC (integrated circuit) card readers; and voice input units such as a microphone and the like.

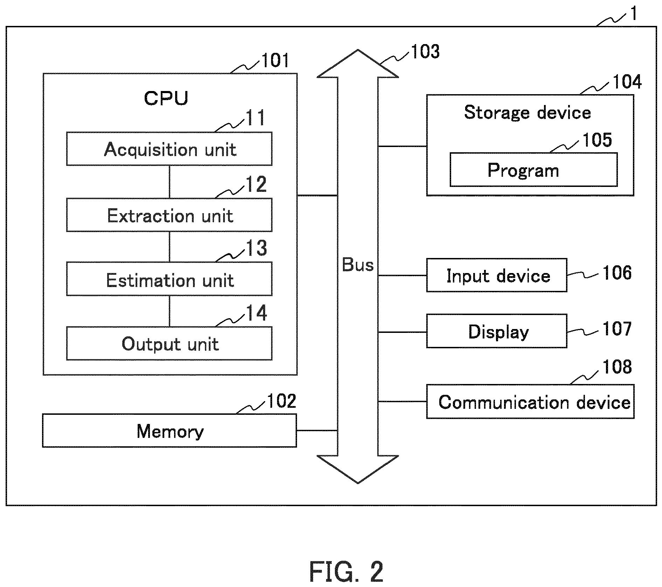

FIG. 2 is a block diagram showing the hardware configuration of the assistance apparatus 1. The assistance apparatus 1 includes, for example, a CPU (central processing unit) 101, a memory 102, a bus 103, a storage device 104, an input device 106, a display 107, a communication device 108, and the like. The respective units of the assistance apparatus 1 are connected with one another via a bus 103 through respective interfaces.

The CPU 101 operates in cooperation with other components by a controller (system controller, I/O controller, or the like), and controls the entire assistance apparatus 1, for example. In the assistance apparatus 1, the program 105 of the present invention and other programs are executed by the CPU 101, and various types of information are read and written, for example. Specifically, for example, the CPU 101 functions as the acquisition unit 11, the extraction unit 12, the estimation unit 13, and the output unit 14. While the assistance apparatus 1 of the present example embodiment includes a CPU as an arithmetic unit, the assistance apparatus 1 may include other arithmetic units such as a GPU (Graphics Processing Unit), an APU (Accelerated Processing Unit), and the like or may include them in combination with the CPU. For example, the CPU 101 functions as each unit other than the storage unit in the second and third example embodiments to be described below.

The memory 102 includes a main memory, for example. The main memory is also referred to as a main memory device. When the CPU 101 performs processing, the memory 102 reads various operation programs such as the program 105 and the like stored in the storage device 104 (auxiliary storage device) to be described below, for example. The CPU 101 then reads the data from the memory 102, decodes the data, and executes the program. The main memory is, for example, a RAM (random access memory). The memory 102 further includes a ROM (read-only memory), for example.

The bus 103 can also be connected to an external device, for example. Examples of the external device include external storage devices (such as an external databases) and printers. The assistance apparatus 1 can be connected to the communication line network 3 by the communication device 108 connected to the bus 103, for example, and can also be connected to the external device via the communication line network 3. The assistance apparatus 1 can also be connected to the assistance terminal 2a, 2b via the communication device 108 and the communication line network 3.

The storage device 104 is also referred to as a so-called auxiliary storage device with respect to the main memory (main memory device), for example. As described above, the storage device 104 stores operation programs including the program 105 of the present invention. The storage device 104 includes a storage medium and a drive for reading from and writing to the storage medium, for example. The storage medium is not particularly limited, and may be, for example, a built-in type or an external type, and examples thereof include HDs (hard disks), FDs (floppy® disks), CD-ROMs, CD-Rs, CD-RWs, MOs, DVDs, flash memories, and memory cards. The drive is not particularly limited. The storage device 104 may be a hard disk drive (HDD) in which the storage medium and the drive are integrated, for example.

The assistance apparatus 1 further includes the input device 106 and the display 107, for example. Examples of the input device 106 include pointing devices such as a touch panel, a track pad, a mouse, and the like; keyboards; imaging units such as a camera, a scanner, and the like; card readers such as an IC card reader, a magnetic card reader, and the like; and voice input units such as a microphone, and the like. Examples of the display 107 include display devices such as an LED display, a liquid crystal display, and the like. While the input device 106 and the display 107 are configured separately in the first example embodiment, the input device 106 and the display 107 may be configured integrally like a touch panel display.

In the assistance apparatus 1, the memory 102 and the storage device 104 can also store access and log information from the user and information acquired from an external database (not shown).

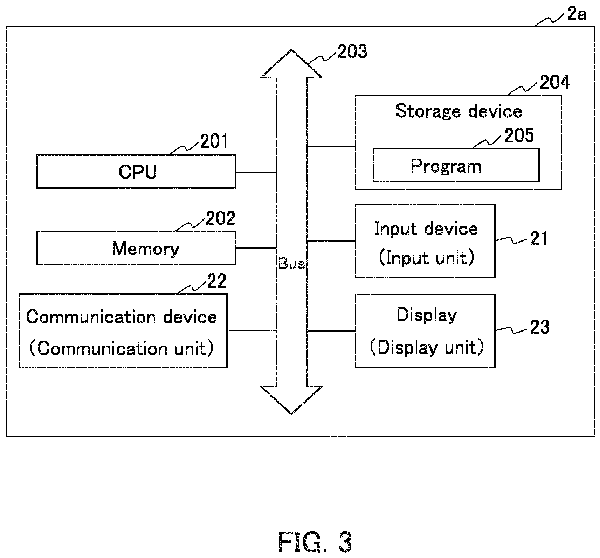

FIG. 3 is a block diagram showing the hardware configuration of the assistance terminal 2a, 2b. The assistance terminal 2a, 2b includes a CPU201, a memory 202, a bus 203, a storage device 204, an input device (input unit) 21, a communication device (communication unit) 22, a display (display unit) 23, and the like, for example. The respective units of the assistance terminal 2a, 2b are connected with one another via a bus 203 through respective interfaces (I/F). Regarding the description of each configuration of the assistance terminal 2a, 2b, reference can be made to the description of each configuration of the assistance apparatus 1.

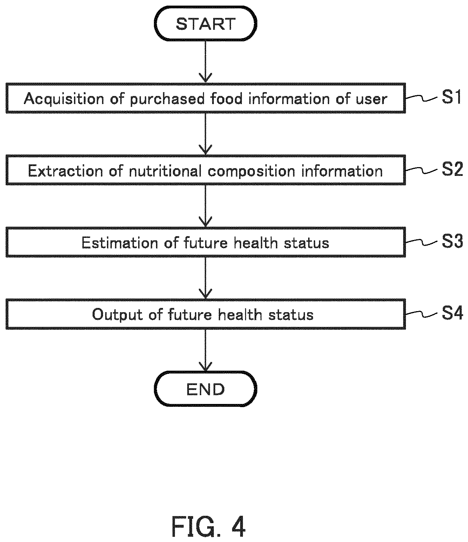

Next, an example of the processing in the assistance apparatus 1 of the present example embodiment will be described with reference to the flow chart of FIG. 4, taking as an example the case of processing based on the purchased food information input from the assistance terminal 2a.

Prior to processing by the assistance apparatus 1, the user first inputs purchased food information of the user by the input unit 21 of the assistance terminal 2a of the user. The user may be, for example, a single user as in the assistance system 100 of the present example embodiment or a plurality of users. The purchased food information is, for example, information on a food purchased by the user. Examples of the purchased food information include a trade name (product name, name) of the purchased food, a manufacturing number, an image of the purchased food, an image of a receipt, an electronic receipt, POS (point of sales) data, image data of a nutrient notation, and date, time, and a location of purchase. Next, the input purchased food information of the user is output to the assistance apparatus 1 via the communication line network 3 by the communication unit 22 of the assistance terminal 2a.

Next, the processing by the assistance apparatus 1 is started. First, the acquisition unit 11 of the assistance apparatus 1 acquires purchased food information output from the assistance terminal 2a via the communication device 108 (step S1, acquisition step). For example, when the extraction unit 12 does not perform extraction from the acquired purchased food information, the acquisition unit 11 may store the purchased food information in the storage device 104.

Next, the extraction unit 12 extracts the nutritional composition information of the purchased food from the purchased food information (step S2, extraction step). When the purchased food information is stored in the storage device 104, the extraction unit 12 may extract the nutritional composition information from the purchased food information stored in the storage device 104, or may extract the nutritional composition information from the purchased food information newly acquired in step S 1 and the purchased food information stored in the storage device 104. The nutritional composition information is, for example, information on a nutritional composition of the purchased food. Examples of the nutritional composition include calories; carbohydrates such as a carbohydrate, a lipid (saturated fatty acid, polyunsaturated fatty acid, cholesterol, etc.), an alcohol, and the like; proteins; inorganic salts such as sodium, calcium, potassium, iron, and the like; dietary fibers such as a poorly soluble dietary fiber, a water-soluble dietary fiber, and the like; Vitamins such as Vitamin A including retinol, α-carotene, β-carotene, β-cryptoxanthin, and the like, Vitamin B family, Vitamin C, Vitamin D, Vitamin E including α-tocopherol, β-tocophenol, γ-tocophenol, σ-tocophenol, and the like, Vitamin K, folic acid, niacin, pantothenic acid, and biotin; and polyphenols. The extraction of the nutritional composition information may be performed, for example, by acquiring nutritional composition information corresponding to the purchased food from a database in or outside the apparatus storing nutritional composition information of each purchased food, or by referring to a food composition reference such as a Standard Tables of Food Composition in Japan for a material constituting each purchased food. When the extraction unit 12 includes purchased food information of different days, for example, it is preferable to extract the nutritional composition information separately for each day. As a specific example, when the purchased food information includes purchased food information for three days, the extraction unit 12 extracts nutritional composition information of the first day based on the purchased food information of the first day, extracts nutritional composition information of the second day based on the purchased food information of the second day, and extracts nutritional composition information of the third day based on the purchased food information of the third day. The extracted nutritional composition information may be stored in the storage device 104, for example.

Next, the estimation unit 13 estimates the future health status of the user (hereinafter also referred to as the "future health status") from the nutritional composition information (step S3, estimation step). The future health status means the health status of the user after the current time. The future health status may be, for example, a health status of the user at any time later than the current time specified by the user, or a health status of the user at a predetermined time later than the current time (e.g., 1 month later, 2 months later, 1 year later, 2 years later, etc.). The future health status may be, for example, one time point or a plurality of time points.

The future health status can be estimated based on, for example, an excessive or insufficient nutritional composition in the nutritional composition information. Specifically, first, the estimation unit 13 calculates a nutritional composition reference value suitable for the user (a nutritional composition reference value of the user), for example, based on the attribute information of the user. The attribute information of the user is acquired by the acquisition unit 11, for example. Examples of the attribute information of the user include the age, sex, height, weight, exercise quantity, METs (METabolic equivalents; is exercise strength indexes), and physical activity levels of the user. The nutritional composition reference value can be calculated from the attribute information of the user and the nutritional composition intake reference for each attribute, for example. Specifically, the nutritional composition reference value can be calculated, for example, by extracting a corresponding nutritional composition value from the nutritional composition intake reference for each attribute based on the attribute information of the user, and integrating the extracted nutritional composition values for each nutritional composition. The nutritional composition reference value is preferably a numerical range. In this case, the nutritional composition reference value may also be referred to as a nutritional composition reference range. The nutritional composition reference range may be set, for example, in the range of ± 15% of the calculated nutritional composition reference value, and preferably in the range of ± 10% or ± 5% of the calculated nutritional composition reference value. When the user is a Japanese, the nutritional composition intake reference for each attribute is, for example, a nutritional composition reference value recommended by the Ministry of Health, Labour and Welfare, and a specific example thereof is the Dietary Reference Intakes for Japanese (2015 edition) (Ministry of Health, Labour and Welfare). When the user is not Japanese, regarding the nutritional composition intake reference for each attribute, for example, reference can be made to the dietary intake references in the country of origin of the user. The reference value may be, for example, a numerical range defined by the lower limit value and the upper limit value.

Next, the estimation unit 13 extracts an excessive or insufficient nutritional composition from the nutritional composition information and the nutritional composition reference value. Specifically, the estimation unit 13 compares the value of each nutritional composition of the nutritional composition information with the corresponding nutritional composition reference value in the nutritional composition reference value, and determines whether the value is less than, equal to (e.g., within the numerical range), or larger than the nutritional composition reference value. Then, the estimation unit 13 determines a nutritional composition that is less than the nutritional composition reference value to be an insufficient nutritional composition, determines a nutritional composition that is equal to the nutritional composition reference value to be a nutritional composition that is not excessive or insufficient, and determines a nutritional composition that is larger than the nutritional composition reference value to be an excessive nutritional composition, for example. The nutritional composition information used by the estimation unit 13 may be, for example, nutritional composition information extracted from purchased food information of 1 day, or may be nutritional composition information extracted from purchased food information of 2 or more days, and is preferably nutritional composition information extracted from purchased food information of about 1 month (around 30 days). In the latter case, preferably, the estimation unit 13 calculates an average value of the nutritional composition information of 1 day based on the nutritional composition information extracted from the purchased food information of 2 days or more, and uses the average value of the nutritional composition information as the nutritional composition information.

Then, the estimation unit 13 estimates the future health status from the excessive or insufficient nutritional composition in the nutritional composition information and the reference composition of the health status. Specifically, as to each excessive or insufficient nutritional composition, the estimation unit 13 determines whether the corresponding excessive or insufficient nutritional composition is present in the reference composition of the health status. Then, when the reference composition of the corresponding health status is present, the estimation unit 13 determines that the future health status is the corresponding health status. On the other hand, when the reference composition of the corresponding health status is not present, the estimation unit 13 determines that the future health status is good. The reference composition of the health status means, for example, an excessive nutritional composition or an insufficient nutritional composition associated with a health status. Examples of the health status include a diseased state of a disease such as undernourishment, dyslipidemia, hypertension, hyperglycemia, sarcopenia, or the like; a state related to a body type such as obesity; and a state not suffering from a disease (a healthy state). Specific examples of the reference composition of the health status include, for example, the following information.

### (Health status: excessive or insufficient nutritional composition)

Hyperglycemia: excess of lipids, proteins, or sugars, or shortage of dietary fiber Dyslipidemia: excess of lipids such as saturated fatty acids, polyunsaturated fatty acids, cholesterol, and the like, sugars, proteins, or alcohols, or shortage of water-soluble dietary fibers Hypertension: excess of sodium (sodium chloride), carbohydrates, alcohols, lipids, or proteins, or shortage of potassium, n-3 fatty acids, or dietary fibers
Sarcopenia: shortage of proteins or shortage of vitamin D or general nutrition

The future health status may be estimated, for example, based on the amount of the nutritional composition in the nutritional composition information. In this case, the estimation unit 13 estimates the future health status based on the amount (intake amount) of the nutritional composition and the relationship between the amount of the nutritional composition and the health status. Specifically, as to the amount of each nutritional composition in the nutritional composition information, the estimation unit 13 compares the information on the relationship between the amount of the nutritional composition and the health status, and extracts the health status corresponding to the amount of the nutritional composition. Then, the estimation unit 13 estimates that the future health status is a health status corresponding to the amount of the nutritional composition. As to the relationship between the amount of the nutritional composition and the health status, reference can be made to known information on the relationship between the food intake amount and the health status. As a specific example, as to the relationship between the salt intake amount (g) and the increase amount in the systolic blood pressure (mmHg) in the year, reference can be made to the following Reference 1.
Reference 1: Intersalt Cooperative Research Group, "Intersalt: an international study of electrolyte excretion and blood pressure. Results for 24 hour urinary sodium and potassium excretion.", BMJ, 1988, volume 297, number 6644, pages 319-328

Next, the output unit 14 outputs the future health status (step S4, output step). Specifically, the output unit 14 outputs the future health status to the assistance terminal 2a via the communication device 108 and the communication line network 3. When there are multiple future health statuses, the output unit 14 may output some or all of them. In the former case, the output unit 14 may output, for example, a predetermined number of future health statuses, or may output a future health status satisfying a condition (e.g., priority) designated by the user. Then, the assistance apparatus 1 terminates the processing. The future health status may be output as, for example, a text, a graph, an image, a character image, or the like, or may be output as instruction information for instructing display of these. The output unit 14 may output the future health status to the assistance terminal 2b of someone other than the user, for example, instead of or in addition to the assistance terminal 2a of the user. The assistance terminal 2b of someone other than the user is, for example, a terminal of an individual or an organization that checks or uses the health status of the user. As a specific example, the terminal 2b may be a terminal of a family member, an insurer, an NPO (Nonprofit Organization) corporation, a volunteer organization, a government, a company, a health insurance association, or the like.

The output future health status is acquired by the assistance terminal 2a via the communication unit 22 of the assistance terminal 2a. Then, the future health status is displayed on the display unit 23 of the assistance terminal 2a.

According to the assistance apparatus, the assistance terminal, and the assistance system of the first example embodiment, the future health status of the user can be estimated and presented from the purchased food information of the user. Thus, according to the assistance apparatus, the assistance terminal, and the assistance system of the first example embodiment, the user can check how the effect of the health management currently performed appears by checking the future health status. Therefore, according to the assistance apparatus, the assistance terminal, and the assistance system of the first example embodiment, it is possible to make it easier for the user to realize health management.

While the estimation unit 13 estimates the future health status in the assistance apparatus, the assistance terminal, and the assistance system of the first example embodiment, the estimation unit 13 may further estimate the current health status of the user from the nutritional composition information. In this case, preferably, the output unit 14 outputs the current health status of the user. The output unit 14 may simultaneously output the future health status and the current health status, or may output them separately. The current health status may be output as, for example, a numerical value (e.g., an excessive value, an insufficient value, and the like) indicating an excessive or insufficient nutritional composition extracted by the estimation unit 13, a diagram (graph or the like) illustrating the numerical value, or the like. The excessive or insufficient nutritional composition can be extracted from the nutritional composition information in the same manner as described above. The nutritional composition information used for estimating the current health status may be, for example, nutritional composition information extracted from purchased food information of 1 day or may be nutritional composition information extracted from purchased food information of 2 or more days, and is preferably nutritional composition information extracted from purchased food information of about 1 month (around 30 days).

### Second example embodiment

The present example embodiment is the same as the first example embodiment except that it includes an image creation unit that creates a character image from the future health status and the character image is output as the future health status. In the present example embodiment, the future health status can be presented to the user as a character image. Thus, according to the present example embodiment, by outputting a health status, which is difficult to disclose to someone other than the user such as a family member or the like, using a character image, it becomes easy to disclose the health status to a third party such as the family member or the like. Therefore, according to the present example embodiment, a third party can intervene in the health management of the user, which promotes the health management of the user.

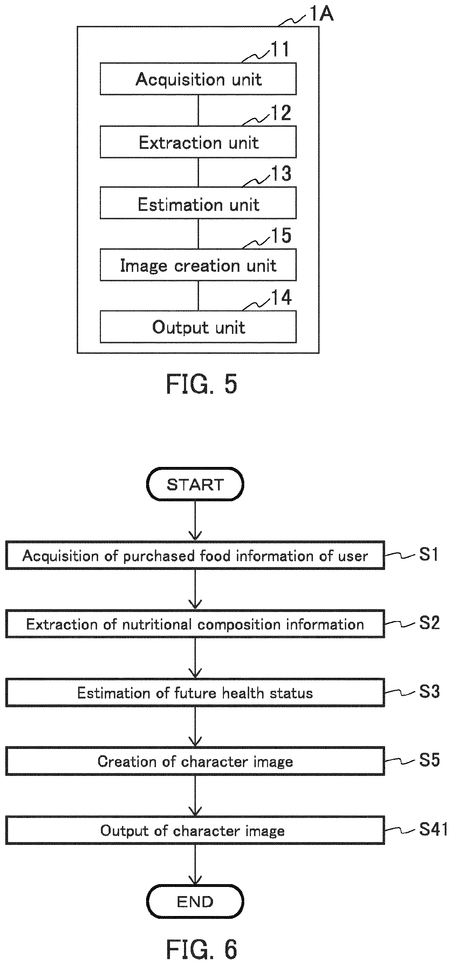

FIG. 5 is a block diagram showing the configuration of an example of an assistance apparatus 1A of the present example embodiment. As shown in FIG. 5, the assistance apparatus 1A further includes an image creation unit 15. The hardware configuration of the assistance apparatus 1A is the same as the first example embodiment except that the CPU 101 includes the configuration of the assistance apparatus 1A of FIG. 5 instead of the configuration of the assistance apparatus 1 of FIG. 1 in the hardware configuration of the assistance apparatus 1 of FIG. 2.

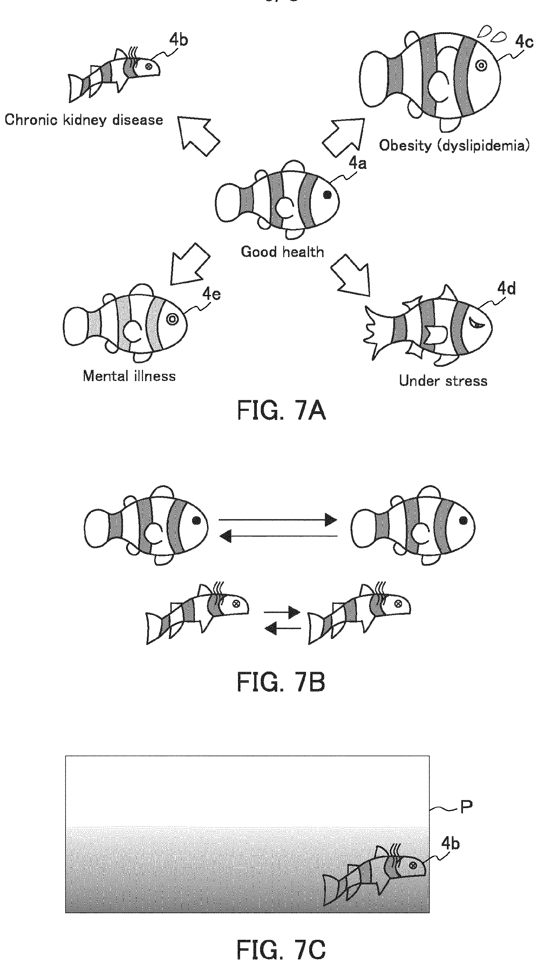

An example of the processing in the assistance apparatus 1A of the present example embodiment will be described with reference to the flow chart of FIG. 6 and the diagram of FIG. 7.

First, steps S1 to S3 are executed in the same manner as steps S1 to S3 in the processing of the assistance apparatus 1 of the first example embodiment.

Next, the image creation unit 15 creates a character image representing an individual of the user from the future health status (step S5, image creation step). The character image may be referred to as an avatar. While the character image may be a human type character image or a non-human type character image, the latter is preferable because the user can more easily accept the future health status. The character image may be created, for example, as shown in the character images 4a, b, c, d, and e of FIG. 7A, by associating the respective health statuses with the character images in advance and extracting the character image of the health status corresponding to the future health status. The character image may be created, for example, by giving a facial expression or the like corresponding to the future health status to a character image designated by the user. When there are multiple future health statuses, the image creation unit 15 may create character images corresponding to them.

Next, the output unit 14 outputs the character image as the future health status (step S41, output step). More specifically, the output unit 14 outputs the future health status to the assistance terminal 2a via the communication device 108 and the communication line network 3. Then, the assistance apparatus 1A terminates the processing. The character image output by the output unit 14 may be information on the character image or information for instructing the display of the character image stored in the assistance terminal 2a of the user, for example.

The output character image is acquired by the assistance terminal 2a via the communication unit 22 of the assistance terminal 2a. The character image is displayed on the display unit 23 of the assistance terminal 2a.

The assistance apparatus 1A of the second example embodiment may change the manner of displaying the character image in accordance with the future health status. As a result, the assistance apparatus 1A of the second example embodiment can more effectively display the future health status of the user. In the case of changing the display of the character image, the assistance apparatus 1A of the second example embodiment may include, for example, a control information acquisition unit that acquires control information for controlling at least one selected from the group consisting of the action of the character (FIG. 7B), the facial expression of the character (FIG. 7A), the background image of the character image (FIG. 7C), and the display position of the character image (FIG. 7C) associated with the health status based on the future health status. The control information is stored in the storage device 104, for example. Thus, the control information acquisition unit acquires control information corresponding to the health information from the storage device 104 based on the future health information, for example. Further, the CPU 101 functions as the control information acquisition unit. Then, the output unit 14 outputs the control information. The control information may be output at the same time as the character image or may be output separately, for example.

The output character image and control information are acquired by the assistance terminal 2a via the communication unit 22 of the assistance terminal 2a. Then, the character image is controlled based on the control information and displayed on the display unit 23 of the assistance terminal 2a.

### Third example embodiment

The present example embodiment is the same as the first example embodiment except that the acquisition unit acquires the attribute information of the user, extracts information on more important nutritional composition depending on the age of the user included in the attribute information of the user, and estimates the future health status of the user based on the extracted nutritional composition. According to the present example embodiment, since information on more important nutritional composition is extracted depending on the age of the user and the future health status of the user is estimated based on the extracted nutritional composition, it is possible to estimate the future health status of the user with better accuracy. Therefore, according to the present example embodiment, it is possible to give the user a sense of trust in the information of the health management, which promotes the health management of the user.

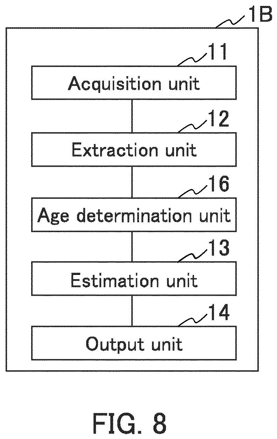

FIG. 8 is a block diagram showing the configuration of an example of an assistance apparatus 1B of the present example embodiment. As shown in FIG. 8, the assistance apparatus 1B further includes an age determination unit 16. The hardware configuration of the assistance apparatus 1B is the same as the first example embodiment except that the CPU 101 includes the configuration of the assistance apparatus 1B of FIG. 8 instead of the configuration of the assistance apparatus 1 of FIG. 1 in the hardware configuration of the assistance apparatus 1 of FIG. 2.

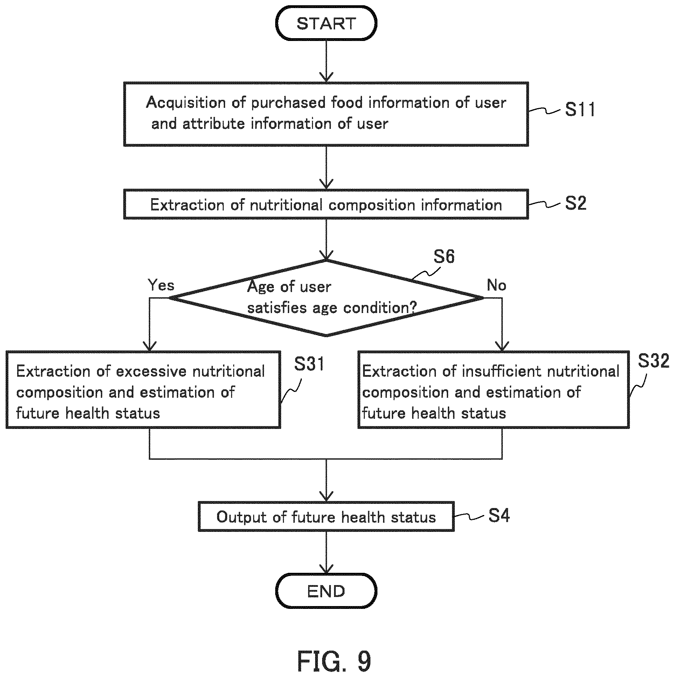

An example of the processing in the assistance apparatus 1B of the present example embodiment will be described with reference to the flow chart of FIG. 9.

First, the acquisition unit 11 acquires the purchased food information of the user and the attribute information of the user (step S11, acquisition step). The purchased food information and the attribute information of the user may be acquired simultaneously or separately, for example. The attribute information of the user may be stored in the storage device 104, for example. In this case, the acquisition unit 11 acquires the attribute information of the user from the storage device 104.

Next, the step S2 is executed in the same manner as the step S2 in the processing of the assistance apparatus 1 of the first example embodiment.

Next, the age determination unit 16 determines whether the age of the user satisfies the age condition (step S6, age determination step). Examples of the age condition include an age separating an elderly and an adult other than the elderly and an age separating an adult and a minor. Specific examples of the age condition include multiple age categories such as 18 to 29 years, 30 to 49 years, 50 to 69 years, 70 years or more, and the like. As to the elderly, for example, a shortage of the nutritional composition is associated with a deterioration in health status. On the other hand, as to the other adult and minor, for example, an excess of the nutritional composition is associated with a deterioration in health status. For this reason, it is preferable to set the age condition such that the elderly proceeds to the step S32 to be described below, and the other adult and minor proceed to the step S31 to be described below.

Then, in the case of Yes, that is, when the age of the user satisfies the age condition, the estimation unit 13 compares the nutritional composition information with the nutritional composition reference value of the user and extracts an excessive nutritional composition in the same manner as in the step S3 in the processing of the assistance apparatus 1 of the first example embodiment (step S31). Then, the estimation unit 13 estimates the future health status of the user from the excessive nutritional composition in the nutritional composition information and the reference composition of the health status. Then, the procedure proceeds to the step S4. On the other hand, in the case of No, that is, when the age of the user does not satisfy the age condition, the estimation unit 13 compares the nutritional composition information with the nutritional composition reference value of the user and extracts an insufficient nutritional composition in the same manner as in the step S3 in the processing of the assistance apparatus 1 of the first example embodiment (step S32). Then, the estimation unit 13 estimates the future health status of the user from the insufficient nutritional composition in the nutritional composition information and the reference composition of the health status.

Next, the step S4 is executed in the same manner as the step S4 in the processing of the assistance apparatus 1 of the first example embodiment, and the processing is terminated.

Fourth example embodiment

The program of the present example embodiment is a program that can execute the health management assistance method on a computer. Alternatively, the program of the present example embodiment may be recorded on, for example, a computer readable recording medium. The recording medium is, for example, a non-transitory computer readable storage medium. The recording medium is not particularly limited, and examples thereof include random access memories (RAMs), read-only memories (ROMs), hard disks (HDs), optical disks, and a floppy® disks (FDs).

While the present invention has been described above with reference to illustrative example embodiments, the present invention is by no means limited thereto. Various changes and variations that may become apparent to those skilled in the art may be made in the configuration and specifics of the present invention without departing from the scope of the present invention.

This application claims priority from Japanese Patent Application No. 2018-156033 filed on August 23, 2018. The entire subject matter of the Japanese Patent Application is incorporated herein by reference.

### (Supplementary Notes)

Some or all of the above example embodiments and examples may be described as in the following Supplementary Notes, but are not limited thereto.

### (Supplementary Note 1)

A health management assistance apparatus, including:
an acquisition unit that acquires purchased food information of a user,
an extraction unit that extracts nutritional composition information of a purchased food from the purchased food information;
an estimation unit that estimates a future health status of the user from the nutritional composition information; and
an output unit that outputs the future health status of the user.

### (Supplementary Note 2)

The health management assistance apparatus according to Supplementary Note 1, including:
an image creation unit that creates a character image representing an individual of the user from the future health status of the user, wherein
the output unit outputs the character image as the future health status of the user.

### (Supplementary Note 3)

The health management assistance apparatus according to Supplementary Note 2, including:
a control information acquisition unit that acquires control information for controlling at least one selected from the group consisting of an action of the character, a facial expression of the character, a background image of the character image, and a display position of the character image associated with the health status based on the future health status of the user, wherein
the output unit outputs the control information.

### (Supplementary Note 4)

The health management assistance apparatus according to any one of Supplementary Notes 1 to 3, wherein
the estimation unit
extracts an excessive or insufficient nutritional composition from the nutritional composition information and a nutritional composition reference value of the user, and
estimates the future health status of the user from the excessive or insufficient nutritional composition in the nutritional composition information and a reference composition of the health status.

### (Supplementary Note 5)

The health management assistance apparatus according to Supplementary Note 4, wherein
the acquisition unit acquires attribute information of the user, and
the estimation unit calculates the nutritional composition reference value of the user from the attribute information of the user and a nutritional composition intake reference value for each attribute.

### (Supplementary Note 6)

The health management assistance apparatus according to any one of Supplementary Notes 1 to 5, wherein
the acquisition unit acquires the attribute information of the user.

### (Supplementary Note 7)

The health management assistance apparatus according to Supplementary Note 6, wherein
the attribute information of the user includes an age of the user,
the apparatus includes an age determination unit that determines whether the age of the user satisfies an age condition,
when the age determination unit determines that the age of the user satisfies the age condition,
the estimation unit
extracts an excessive nutritional composition by comparing the nutritional composition information with the nutritional composition reference value of the user, and
estimates the future health status of the user from the excessive nutritional composition in the nutritional composition information and a reference composition of a health status.

### (Supplementary Note 8)

The health management assistance apparatus according to Supplementary Note 6 or 7, wherein the attribute information of the user includes an age of the user,
the apparatus includes an age determination unit that determines whether the age of the user satisfies an age condition,
when the age determination unit determines that the age of the user does not satisfy the age condition,
the estimation unit
extracts an insufficient nutritional composition by comparing the nutritional composition information with the nutritional composition reference value of the user, and
estimates the future health status of the user from the insufficient nutritional composition in the nutritional composition information and a reference composition of a health status.

### (Supplementary Note 9)

The health management assistance apparatus according to any one of Supplementary Notes 1 to 8, wherein
the estimation unit estimates a current health status of the user from the nutritional composition information.

### (Supplementary Note 10)

The health management assistance apparatus according to Supplementary Note 9, wherein
the output unit outputs the current health status of the user.

### (Supplementary Note 11)

The health management assistance apparatus according to any one of Supplementary Notes 1 to 10, wherein
the output unit outputs the future health status of the user to a terminal of the user.

### (Supplementary Note 12)

The health management assistance apparatus according to any one of Supplementary Notes 1 to 10, wherein
the output unit outputs the future health status of the user to a terminal different from a terminal of the user.

### (Supplementary Note 13)

The health management assistance apparatus according to any one of Supplementary Notes 1 to 12, wherein
the extraction unit acquires the nutritional composition information of the purchased food from outside the apparatus.

### (Supplementary Note 14)

The health management assistance apparatus according to any one of Supplementary Notes 1 to 13, which is a server.

### (Supplementary Note 15)

A health management assistance method, including:
an acquisition step of acquiring purchased food information of a user,
an extraction step of extracting nutritional composition information of a purchased food from the purchased food information;
an estimation step of estimating a future health status of the user from the nutritional composition information; and
an output step of outputting the future health status of the user.

### (Supplementary Note 16)

The health management assistance method according to Supplementary Note 15, including:
an image creation step of creating a character image representing an individual of the user from the future health status of the user, wherein
in the output step, the character image is output as the future health status of the user.

### (Supplementary Note 17)

The health management assistance method according to Supplementary Note 16, including:
a control information acquisition step of acquiring control information for controlling at least one selected from the group consisting of an action of the character, a facial expression of the character, a background image of the character image, and a display position of the character image associated with the health status based on the future health status of the user, wherein
in the output step, the control information is output.

### (Supplementary Note 18)

The health management assistance method according to any one of Supplementary Notes 15 to 17, wherein
in the estimation step
an excessive or insufficient nutritional composition is extracted from the nutritional composition information and a nutritional composition reference value of the user, and
the future health status of the user is estimated from the excessive or insufficient nutritional composition in the nutritional composition information and a reference composition of a health status.

### (Supplementary Note 19)

The health management assistance method according to Supplementary Note 18, wherein
in the acquisition step, attribute information of the user is acquired, and
in the estimation step, the nutritional composition reference value of the user is calculated from the attribute information of the user and a nutritional composition intake reference value for each attribute.

### (Supplementary Note 20)

The health management assistance method according to any one of Supplementary Notes 15 to 19, wherein
in the acquisition step, the attribute information of the user is acquired.

### (Supplementary Note 21)

The health management assistance method according to Supplementary Note 20, wherein
the attribute information of the user includes an age of the user,
the method includes an age determination step of determining whether the age of the user satisfies an age condition,
when the age of the user is determined to satisfy the age condition in the age determination step,
in the estimation step,
an excessive nutritional composition is extracted from the nutritional composition information and the nutritional composition reference value of the user, and
the future health status of the user is estimated from the excessive nutritional composition in the nutritional composition information and a reference composition of a health status.

### (Supplementary Note 22)

The health management assistance method according to Supplementary Note 20 or 21, wherein the attribute information of the user includes an age of the user,
the method includes an age determination step of determining whether the age of the user satisfies an age condition,
when the age of the user is determined not to satisfy the age condition in the age determination step,
in the estimation step
an insufficient nutritional composition is extracted from the nutritional composition information and the nutritional composition reference value of the user, and
the future health status of the user is estimated from the insufficient nutritional composition in the nutritional composition information and a reference composition of a health status.

### (Supplementary Note 23)

The health management assistance method according to any one of Supplementary Notes 15 to 22, wherein
in the estimation step, a current health status of the user is estimated from the nutritional composition information.

### (Supplementary Note 24)

The health management assistance method according to Supplementary Note 23, wherein
in the output step, the current health status of the user is output.

### (Supplementary Note 25)

The health management assistance method according to any one of Supplementary Notes 15 to 24, wherein
in the output step, the future health status of the user is output to a terminal of the user.

### (Supplementary Note 26)

The health management assistance method according to any one of Supplementary Notes 15 to 24, wherein
in the output step, the future health status of the user is output to a terminal different from a terminal of the user.

### (Supplementary Note 27)

The health management assistance method according to any one of Supplementary Notes 15 to 26, wherein
in the extraction step, the nutritional composition information of the purchased food is acquired from outside the apparatus.

### (Supplementary Note 28)

A health management assistance terminal, including:
an input unit that inputs purchased food information of a user;
a communication unit configured to communicate with the health management assistance apparatus according to any one of Supplementary Notes 1 to 14 that estimates a future health status of the user from the purchased food information of the user; and
a display unit that displays the future health status of the user.

### (Supplementary Note 29)

A program that can execute the following processing on a computer:
acquisition processing of acquiring purchased food information of a user;
extraction processing of extracting nutritional composition information of the purchased food from the purchased food information;
estimation processing of estimating a future health status of the user from the nutritional composition information; and
output processing of outputting the future health status of the user.

### (Supplementary Note 30)

A computer readable recording medium with the program according to Supplementary Note 29.

### (Supplementary Note 31)

A health management assistance system, including:
a terminal; and
a server, wherein
the terminal and the server are connectable via a communication line network outside the system,
the terminal includes:
   an input unit that inputs purchased food information of a user;
   a communication unit configured to communicate with the server; and
   a display unit that displays a future health status of the user, and
the server includes:
   an acquisition unit that acquires the purchased food information of the user;
   an extraction unit that extracts nutritional composition information of the purchased food from the purchased food information;
   an estimation unit that estimates the future health status of the user from the nutritional composition information; and
   an output unit that outputs the future health status of the user.

### Industrial Applicability

According to the present invention, the future health status of the user can be estimated and presented from the purchased food information of the user. Therefore, according to the present invention, the user can check how the effect of the health management currently performed appears by checking the future health status. Thus, according to the present invention, it is possible to make it easier for the user to realize health management. Accordingly, the present invention is extremely useful in the medical field, the preventive medical field, and the like. Reference Signs List

- 1, 1A, 1B:: assistance apparatus
- 11:: acquisition unit
- 12:: extraction unit
- 13:: estimation unit
- 14:: output unit
- 15:: image creation unit
- 16:: age determination unit
- 101,201:: CPU
- 102,202:: memory
- 103,203:: bus
- 104, 204:: storage device
- 105,205:: program
- 106:: input device
- 107:: display
- 108:: communication device
- 2a, 2b:: assistance terminal
- 21:: input unit
- 22:: communication unit
- 23:: display unit
- 3:: communication line network
- 4a, b, c, d, e:: character image

## Claims

1. A health management assistance apparatus, comprising:
an acquisition unit that acquires purchased food information of a user,
an extraction unit that extracts nutritional composition information of a purchased food from the purchased food information;
an estimation unit that estimates a future health status of the user from the nutritional composition information; and
an output unit that outputs the future health status of the user.

2. The health management assistance apparatus according to claim 1, comprising:
an image creation unit that creates a character image representing an individual of the user from the future health status of the user, wherein
the output unit outputs the character image as the future health status of the user.

3. The health management assistance apparatus according to claim 2, comprising:
a control information acquisition unit that acquires control information for controlling at least one selected from the group consisting of an action of the character, a facial expression of the character, a background image of the character image, and a display position of the character image associated with the health status based on the future health status of the user, wherein
the output unit outputs the control information.

4. The health management assistance apparatus according to any one of claims 1 to 3, wherein
the estimation unit
extracts an excessive or insufficient nutritional composition from the nutritional composition information and a nutritional composition reference value of the user, and
estimates the future health status of the user from the excessive or insufficient nutritional composition in the nutritional composition information and a reference composition of the health status.

5. The health management assistance apparatus according to claim 4, wherein
the acquisition unit acquires attribute information of the user, and
the estimation unit calculates the nutritional composition reference value of the user from the attribute information of the user and a nutritional composition intake reference value for each attribute.

6. The health management assistance apparatus according to any one of claims 1 to 5, wherein
the acquisition unit acquires the attribute information of the user.

7. The health management assistance apparatus according to claim 6, wherein
the attribute information of the user includes an age of the user,
the apparatus comprises an age determination unit that determines whether the age of the user satisfies an age condition,
when the age determination unit determines that the age of the user satisfies the age condition,
the estimation unit
extracts an excessive nutritional composition by comparing the nutritional composition information with the nutritional composition reference value of the user, and
estimates the future health status of the user from the excessive nutritional composition in the nutritional composition information and a reference composition of a health status.

8. The health management assistance apparatus according to claim 6 or 7, wherein
the attribute information of the user includes an age of the user,
the apparatus comprises an age determination unit that determines whether the age of the user satisfies an age condition,
when the age determination unit determines that the age of the user does not satisfy the age condition,
the estimation unit
extracts an insufficient nutritional composition by comparing the nutritional composition information with the nutritional composition reference value of the user, and
estimates the future health status of the user from the insufficient nutritional composition in the nutritional composition information and a reference composition of a health status.

9. The health management assistance apparatus according to any one of claims 1 to 8, wherein
the estimation unit estimates a current health status of the user from the nutritional composition information.

10. The health management assistance apparatus according to claim 9, wherein
the output unit outputs the current health status of the user.

11. The health management assistance apparatus according to any one of claims 1 to 10, wherein
the output unit outputs the future health status of the user to a terminal of the user.

12. The health management assistance apparatus according to any one of claims 1 to 10, wherein
the output unit outputs the future health status of the user to a terminal different from a terminal of the user.

13. The health management assistance apparatus according to any one of claims 1 to 12, wherein
the extraction unit acquires the nutritional composition information of the purchased food from outside the apparatus.

14. The health management assistance apparatus according to any one of claims 1 to 13, which is a server.

15. A health management assistance method, comprising:
an acquisition step of acquiring purchased food information of a user,
an extraction step of extracting nutritional composition information of a purchased food from the purchased food information;
an estimation step of estimating a future health status of the user from the nutritional composition information; and
an output step of outputting the future health status of the user.

16. The health management assistance method according to claim 15, comprising:
an image creation step of creating a character image representing an individual of the user from the future health status of the user, wherein
in the output step, the character image is output as the future health status of the user.

17. The health management assistance method according to claim 16, comprising:
a control information acquisition step of acquiring control information for controlling at least one selected from the group consisting of an action of the character, a facial expression of the character, a background image of the character image, and a display position of the character image associated with the health status based on the future health status of the user, wherein
in the output step, the control information is output.

18. The health management assistance method according to any one of claims 15 to 17, wherein
in the estimation step
an excessive or insufficient nutritional composition is extracted from the nutritional composition information and a nutritional composition reference value of the user, and
the future health status of the user is estimated from the excessive or insufficient nutritional composition in the nutritional composition information and a reference composition of a health status.

19. The health management assistance method according to claim 18, wherein
in the acquisition step, attribute information of the user is acquired, and
in the estimation step, the nutritional composition reference value of the user is calculated from the attribute information of the user and a nutritional composition intake reference value for each attribute.

20. The health management assistance method according to any one of claims 15 to 19, wherein
in the acquisition step, the attribute information of the user is acquired.

21. The health management assistance method according to claim 20, wherein
the attribute information of the user includes an age of the user,
the method comprises an age determination step of determining whether the age of the user satisfies an age condition,
when the age of the user is determined to satisfy the age condition in the age determination step,
in the estimation step,
an excessive nutritional composition is extracted from the nutritional composition information and the nutritional composition reference value of the user, and
the future health status of the user is estimated from the excessive nutritional composition in the nutritional composition information and a reference composition of a health status.

22. The health management assistance method according to claim 20 or 21, wherein
the attribute information of the user includes an age of the user,
the method comprises an age determination step of determining whether the age of the user satisfies an age condition,
when the age of the user is determined not to satisfy the age condition in the age determination step,
in the estimation step
an insufficient nutritional composition is extracted from the nutritional composition information and the nutritional composition reference value of the user, and
the future health status of the user is estimated from the insufficient nutritional composition in the nutritional composition information and a reference composition of a health status.

23. The health management assistance method according to any one of claims 15 to 22, wherein
in the estimation step, a current health status of the user is estimated from the nutritional composition information.

24. The health management assistance method according to claim 23, wherein
in the output step, the current health status of the user is output.

25. The health management assistance method according to any one of claims 15 to 24, wherein
in the output step, the future health status of the user is output to a terminal of the user.

26. The health management assistance method according to any one of claims 15 to 24, wherein
in the output step, the future health status of the user is output to a terminal different from a terminal of the user.

27. The health management assistance method according to any one of claims 15 to 26, wherein
in the extraction step, the nutritional composition information of the purchased food is acquired from outside the apparatus.

28. A health management assistance terminal, comprising:
an input unit that inputs purchased food information of a user;
a communication unit configured to communicate with the health management assistance apparatus according to any one of claims 1 to 14 that estimates a future health status of the user from the purchased food information of the user; and
a display unit that displays the future health status of the user.

29. A program that can execute the following processing on a computer:
acquisition processing of acquiring purchased food information of a user;
extraction processing of extracting nutritional composition information of the purchased food from the purchased food information;
estimation processing of estimating a future health status of the user from the nutritional composition information; and
output processing of outputting the future health status of the user.

30. A computer readable recording medium with the program according to claim 29.

31. A health management assistance system, comprising:
a terminal; and
a server, wherein
the terminal and the server are connectable via a communication line network outside the system,
the terminal comprises:
an input unit that inputs purchased food information of a user;
a communication unit configured to communicate with the server; and
a display unit that displays a future health status of the user, and
the server comprises:
an acquisition unit that acquires the purchased food information of the user;
an extraction unit that extracts nutritional composition information of the purchased food from the purchased food information;
an estimation unit that estimates the future health status of the user from the nutritional composition information; and
an output unit that outputs the future health status of the user.
